# EUROPEAN PATENT SPECIFICATION

(11) **EP 0 955 983 B1**
(45) Date of publication and mention of the grant of the patent: **02.05.2003**
(21) Application number: 97953071.4
(22) Date of filing: 02.12.1997
(51) Int. Cl.: A61F 15/00, A61M 35/00

(54) **TRANSDERMAL/TRANSMUCOSAL PATCH DISPENSER**
SPENDER FÜR TRANSDERMALE/TRANSMUCOSALE PFLASTER
DISTRIBUTEURS DE TIMBRES TRANSDERMIQUES/TRANSMUQUEUX

(30) Priority: 03.12.1996 US 759898
(43) Date of publication of application: 17.11.1999
(73) Proprietor: MINNESOTA MINING AND MANUFACTURING COMPANY, St. Paul, Minnesota 55133-3427 (US)
(72) Inventor: SITZ, Richard, G., Saint Paul, MN 55133-3427 (US); COOKLOCK, Kathleen, M., Saint Paul, MN 55133-3427 (US); PETERSON, Kenneth, A., Saint Paul, MN 55133-3427 (US); ROBISON, Thomas, S., Saint Paul, MN 55133-3427 (US); SEVER, John, M., Saint Paul, MN 55133-3427 (US); WHITMAN, David, A., Saint Paul, MN 55133-3427 (US)
(74) Representative: VOSSIUS & PARTNER
(86) International application number: US9721971
(87) International publication number: WO98024393

(56) References cited:
- EP-A- 0 422 922
- WO-A-95/18046
- US-A- 3 520 403
- US-A- 3 835 992
- US-A- 4 627 429
- US-A- 4 993 586
- US-A- 5 358 140

## Description

The present invention relates to transdermal/transmucosal drug delivery patches and, in particular, to dispensers for holding and dispensing such patches.

Transdermal and transmucosal patches containing drugs such as estradiol, levonorgestral, testosterone, scopolamine, nitroglycerin, nicotine, heparin, melatonin, diagnostic compounds, and other ingredients are conventionally required to be packaged in hermetically sealed, individual foil and/or plastic pouches. Such packaging is necessary in order to maintain sanitary conditions and to prevent degradation, contamination, and/or loss of sensitive ingredients by environmental exposure. In many cases, the material used for such packaging has to be sufficiently impervious to volatile active ingredients, e.g., nitroglycerin, and environmental factors, e.g., humidity, to allow the device to remain stable under extended storage conditions of up to several years.

However, a serious disadvantage of the conventional packaging is that the user must tear open a separate pouch each time one of the transdermal or transmucosal patches is needed. These pouches can be difficult to open, particularly for the elderly and those with conditions that impair strength and/or manual dexterity, and especially if the packaging is made of tear resistant or hard to grasp materials. Even for those without any special difficulties, opening such pouches on a frequent basis over an extended period can be a substantial inconvenience.

Moreover, individual packaging can be expensive and wasteful due to the excessive packaging material required and manufacturing cost for individual packaging. In some cases each individual pouch not only contains an individual transdermal or transmucosal drug containing patch, but also must include desiccant to prevent moisture from affecting the patch or active ingredient contained therein, thereby further increasing costs.

WO-A-95/18046 discloses an encased adhesive-coated article comprising a continuous support member, an adhesive-coated article including a first adhesive surface and a cover member. The first adhesive surface has a first adhesive coating covering at least a portion thereof. The adhesive coated article is removably adhered to the support member by contact of the first adhesive coating, wherein the first adhesive coating between the support member and the first adhesive surface forms a first adhesive bond. The cover member is removably attached to the support member to releaseably encase the adhesive-coated article. The cover member further includes a second adhesive coating covering at least a portion thereof, wherein the cover member is removably attached to the support member by contact of the second adhesive coating with the support member, the contact between the support member and the cover member forming a second adhesive bond. The articles of WO-A-95/18046 are dispensed by separation from the continuous support member and peeling them off therefrom.

EP-A-0 422 922 discloses a pop-up dispenser containing bandages such as Band-Aid™ Brand Sheer Strips. This document does not disclose or suggest in any way using the dispensing apparatus for dispensing transdermal/transmucosal drug delivery patches.

It would thus be highly desirable to have a way of packaging transdermal and transmucosal drug-containing patches that is sanitary, simple and easy to open, economical, and sufficiently impervious to prevent loss of volatile components and protect the patches from environmental exposure.

The present invention as defined in the claims addresses the above problems, as well as others that will become apparent upon reference to the accompanying drawings and following detailed description, by providing a dispenser for conveniently dispensing multiple transdermal/transmucosal drug-containing patches from a single container. The dispenser, which is preferably made of an impermeable material (e.g., plastic, as opposed to mere cardboard), has a sealed chamber containing a plurality of individual patches, and at least one aperture through which the patches can be removed from the dispenser. The transdermal/transmucosal patches are arranged such that the removal of one patch thereby allows the next patch to be grasped and removed, and preferably the patches are separably interconnected together such that the action of removing one of the transdermal/transmucosal patches thereby moves a next one of the patches into position for subsequent removal.

In one preferred embodiment, the dispenser walls defining the chamber are made of rigid plastic and the aperture is in the form of a single slot through which each of the transdermal/transmucosal patches is pulled. Each patch is preferably separably interconnected to an adjacent patch so that pulling one patch out of the slot pulls a portion of the next patch through the slot and into position for subsequent grasping and removal. The separable interconnection of the patches is preferably accomplished in one of essentially three ways: (1) via a continuous release liner upon which the individual transdermal/transmucosal patches are commonly adhered, in which case the liner is preferably perforated between each patch to facilitate separation, (2) without a continuous release liner, but where there is either an added portion of adhesive between the release liner and backing of successive patches or where a part of the adhesive underside of one patch is directly adhered to a portion of the top surface of the next patch to be dispensed, or (3) without a continuous release liner, but where a portion of each patch is interleaved beneath a portion of the next patch to be dispensed.

The patches, which generally are not individually packaged, may be folded, stacked, and/or interleaved together one on top of another within the chamber so that a large number of patches can be stored efficiently in a small space. Alternatively, the individual transdermal/transmucosal patches may not be folded, stacked, and/or interleaved on top of each other, but instead arranged sequentially on a roll, or on a flat strip or continuous loop arrangement. In this case, depending on the design, the dispenser may have either a single aperture or multiple apertures (one for each patch). Also, the aperture may include a resilient sealing member to help prevent escape of volatile components and/or environmental contamination of the transdermal/transmucosal patch-containing chamber.

The dispenser may also preferably include a cover that can be opened to access and remove one or more of the transdermal or transmucosal drug-containing patches and then closed to prevent contamination. The cover preferably provides an impermeable seal, particularly in situations where loss of volatile components and/or environmental exposure of the remaining patches may be a problem. Moreover, the dispenser may contain a desiccant to help further prevent exposure to moisture if necessary, and may also include a moisture exposure indicator strip to monitor whether or not the transdermal/transmucosal patches have been exposed to an unacceptable amount of moisture during storage.

To further protect the transdermal/transmucosal patches, and provide for extended storage, it will be preferable in some cases to package the multi-patch dispenser itself in an impermeable packaging material, such as a foil and/or polymer pouch. In this case, the impermeable dispenser packaging provides a sufficiently impermeable barrier for longer term storage until opened, after which the multi-patch dispenser itself provides sufficient short/medium term protection of the transdermal/transmucosal patches.

The present invention also greatly facilitates the use of non-uniform transdermal/transmucosal drug-containing patches because the patches are preferably dispensed one by one in a specific predetermined order. For example, it may be desirable in some situations to vary doses of an active ingredient over the course of a certain time period (e.g., a diminishing dose of nicotine; or varying the amount of a hormone, such as estrogen, over the course of a monthly cycle). This can very simply and reliably be accomplished with the present invention by, for example, making the size of or the concentration of active ingredient in the patch vary as desired within the dispenser. Similarly, it may also be desirable in some cases to provide a marking -- such as numbering or days of the week -- in association with the transdermal/transmucosal patches in order to help the user adhere to the proper treatment regimen. Again, this is facilitated by the present invention since the patches will normally be dispensed in a predetermined sequence.

In a further aspect of the invention, the dispenser may include a ratcheting mechanism so that only one medical adhesive patch at a time can be removed. This is particularly convenient where a continuous release liner is used, since the ratcheting mechanism will prevent more than one of the transdermal/transmucosal patches from being accidentally withdrawn and will also allow for easier separation by simply pulling on the patch being removed. Further, the dispenser may contain a counter mechanism that is actuated by the removal of a patch in order to indicate such things as the number of patches used or the number remaining in the container, or to display a corresponding day of the week, and so on. The counter mechanism may or may not be integrated as part of a ratcheting mechanism.

Preferred drug-containing transdermal/transmucosal patches to be dispensed from a dispenser according to the present invention are those which are administered on a weekly, twice-weekly, or daily basis, such as hormone replacement therapy and fertility control patches containing, for example, estradiol, progestin, and/or testosterone. Other examples of preferred patches include frequently applied patches such as nitroglycerin, melatonin, heparin, calcitonin, prostaglandin E₁-ethyl ester, glucose monitoring, and insulin containing patches. Also, as noted above, the present invention is particularly advantageous for use with non-uniform transdermal/transmucosal patches that are to be administered in a particular predetermined sequence, for example where the dose of drug to be delivered varies over the treatment period. As used herein, the term drug refers to a substance intended for use in the diagnosis, cure, mitigation, treatment or prevention of disease, or to affect the structure or function of the body. In the present transdermal/transmucosal context, the drug is preferably for systemic delivery. When used for diagnostic purposes, the drug may be contained within the patch and bodily fluids transdermally/transmucosally absorbed into the patch for reaction with the drug, such as in the case of glucose monitoring (see, e.g., U.S. Patent 5,113,860).

Finally, the present invention also provides a method of packaging transdermal/transmucosal drug-containing patches for convenient dispensing, comprising the steps of (i) providing a dispenser unit having walls defining a chamber and an aperture, and (ii) packaging a plurality of transdermal/transmucosal drug-containing patches within the chamber and, preferably, arranging the patches so as to allow (and preferably require) removal of one patch at a time in a predetermined sequential order through the aperture.

Preferred embodiments of the invention will now be described in greater detail below with reference to the attached drawings, wherein:
FIG. 1 is a perspective view of a transdermal/transmucosal patch dispenser unit with a closed lid;
FIG. 2 is a perspective view of a dispenser unit containing multiple stacked transdermal/transmucosal patches, with an open lid and a transdermal/transmucosal patch partially exposed;
FIG. 3a is a cross-section along the line 3-3 of FIG. 2 showing a particular transdermal/transmucosal patch stacking embodiment;
FIG. 3b is a cross-section along the line 3-3 of FIG. 2 showing an alternative stacking embodiment;
FIG. 4 is a perspective view of a dispenser unit - not falling within the scope of the claims - containing multiple folded transdermal/transmucosal patches, with an open lid and a patch partially exposed;
FIG. 5 is a cross-section along the line 5-5 of FIG. 4;
FIG. 6 is a perspective view of the dispenser of FIG. 5 with a patch in position for separation;
FIG. 7 is a cross-sectional side view of a roll type embodiment not falling within the scope of the claims;
FIG. 8 is a cross-sectional side view of a multi-slot embodiment not falling within the scope of the claims;
FIG. 9 is a cross-sectional side view of continuous loop embodiment not falling within the scope of the claims; and
FIG. 10 is a perspective view of a transdermal/transmucosal patch dispenser contained in an impermeable storage pouch.

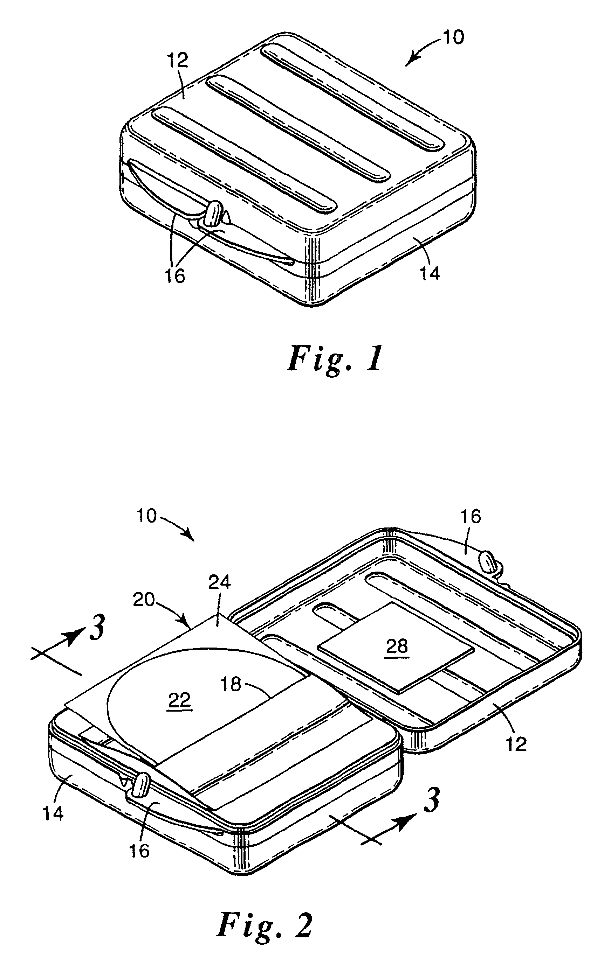

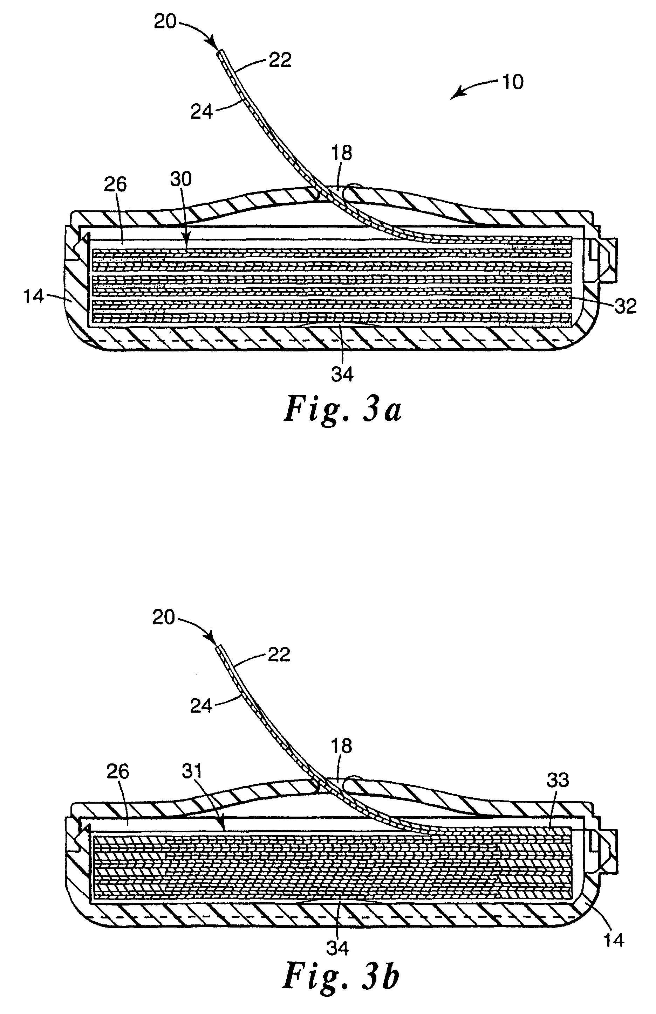

FIG. 1 shows a dispenser 10 according to a preferred embodiment of the invention. The dispenser 10 includes a cover 12, a base portion 14, and latching flanges 16. As best seen in FIG. 2 with the cover 12 open, the base portion 14 includes a slot 18 through which a transdermal/transmucosal drug-containing patch 20 can be removed from a chamber 26 (best seen in FIG. 3). The cover 12 and base portion 14 of the dispenser are preferably formed of a rigid plastic material.

The cover 12 and base portion 14 are preferably made so that, when closed, as in FIG. 1, the seal formed between the two is sufficiently impermeable to prevent substantial losses of volatile components of the transdermal/transmucosal patches and/or degradation or contamination from environmental factors, such as humidity, microbes, and the like. This can be accomplished by means such as using elastomeric seal materials along the mating edges of the cover 12 and base portion 14. The latching flanges 16 allow the base portion 14 and cover 12 to be securely snapped together, yet easily opened when access is desired. The dispenser 10 may also include a contamination indicator 28 which indicates, for example, the degree of moisture exposure of the interior of the dispenser.

As shown in FIG. 2, a portion of the transdermal/transmucosal drug-containing patch 20 extends through slot 18. The transdermal/transmucosal patch 20 has been shown, for convenience, as only two layers, a release liner 24 and an adhesive/backing layer 22. It will be understood that the adhesive/backing layer 22 of transdermal/transmucosal patch will typically be composed of two or more layers, including a backing layer and at least one adhesive layer. The drug in the transdermal/transmucosal patches may be in a reservoir or mixed into at least one of the layers of adhesive/backing layer 22. Excipients, such as penetration enhancers and crystallization inhibitors, may also be included. Also, it should be noted that there is no requirement that the transdermal/transmucosal patches be uniform, layered transdermal/transmucosal patches such as those shown. For example, the patches may vary in size or shape and may include various structures, such as drug reservoirs.

When grasped and pulled by a user, the transdermal/transmucosal drug-containing patch 20 can be removed from the dispenser and a next transdermal/transmucosal patch 30, shown in FIG. 3a, will be concurrently pulled into place with a portion thereof extending out through slot 18. This is accomplished in the embodiment of FIG. 3a by having each of the individual patches separably interconnected by a small portion of adhesive 32 located adjacent alternating ends of the patches. The adhesive is sufficient so that when transdermal/transmucosal patch 20 is pulled through slot 18 the end portion of the next transdermal/transmucosal patch 30 is also pulled through slot 18. The adhesive portion 32 then releases (due to changes in the angle of force acting on the adhesive portion) and the patch 20 is thereby separated from the next patch 30.

FIG. 3b is similar to FIG. 3a and operates in substantially the same manner. However, rather than having an additional adhesive portion 32 between successive transdermal/transmucosal patches, as in FIG. 3a, the embodiment of FIG. 3b has an area 33 near one end of the transdermal/transmucosal patch 21 where the release liner 24 is absent and the adhesive/backing layer 22 adheres directly to the backing of the next succeeding transdermal/transmucosal patch 31.

In some cases, it may be preferable for the dispenser to include a desiccant material 34, which may be located in the transdermal/transmucosal patch containing chamber 26 (as shown) or elsewhere, such as under the cover. The desiccant helps to prevent the patches from being contaminated/degraded by exposure to moisture. It also may be preferable in some cases to provide a resilient seal (not shown) along slot 18 to further prevent the egress of volatile components or ingress of contaminants.

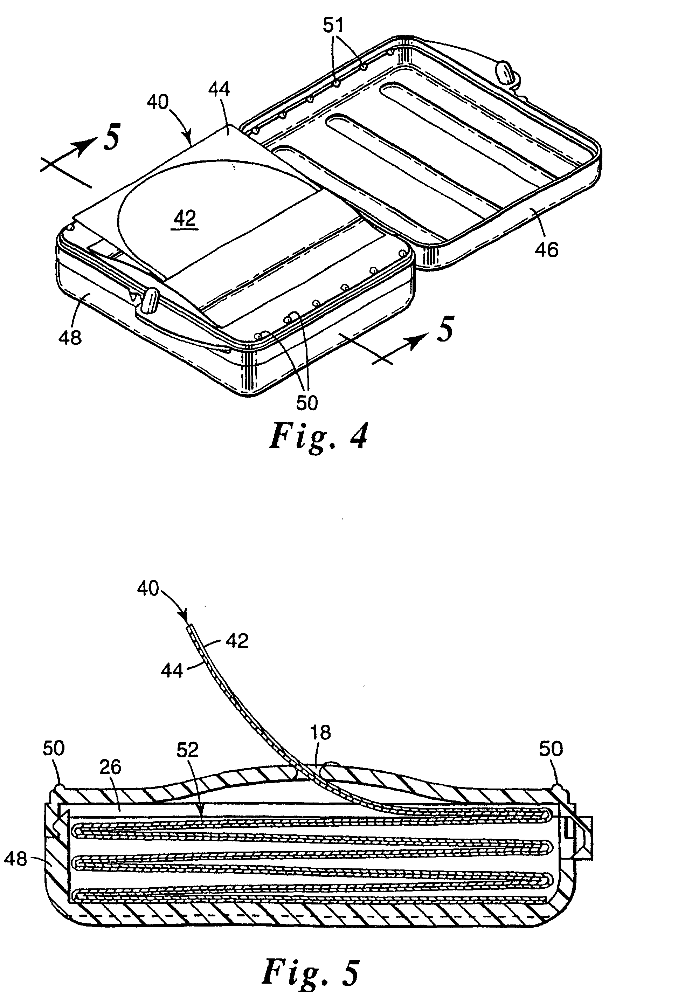

Turning now to FIGS. 4-6, the dispenser shown differs from the above-described embodiment in that the plurality of transdermal/transmucosal patches are separably interconnected by a continuous release liner 44, rather than by adhesive portions. As seen in FIG. 5, the individual transdermal/transmucosal patches are folded together in alternating directions on top of one another. The release liner is preferably perforated (not shown) between each individual patch in order to facilitate separation. Also, since a continuous release liner is used, it would be possible to pull more than one of the patches out of the dispenser at a time. If this is not desired, which will usually be the case, the successive transdermal/transmucosal patches must be restrained while the desired terminal patch 40 is separated. This can be accomplished manually (not shown), by simply holding the exposed portion of the next succeeding transdermal/transmucosal patch 52 after the patch 40 is pulled completely out of the slot 18.

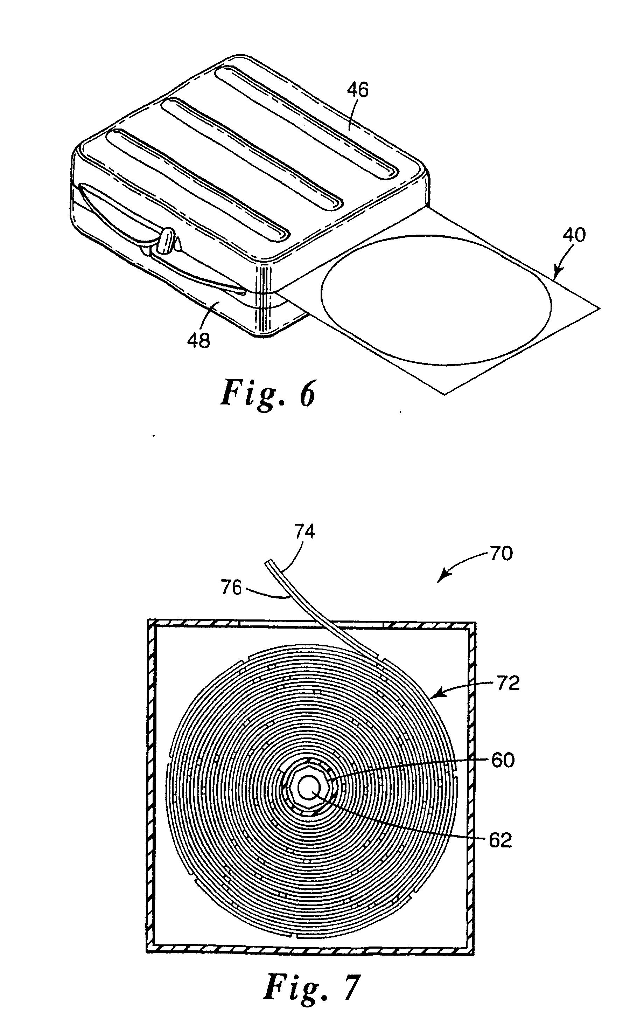

Alternatively, it is possible to size the dispenser cover 46 and base portion 48 relative to the transdermal/transmucosal patches such that when the patch 40 is pulled out, thereby drawing with it the next patch 52, it can be positioned so that, as shown in FIG. 6, the cover 46 can be closed in order to hold the next patch 52 (not visible in FIG. 6) and allow separation of the desired patch 40 extending outside of the dispenser. To facilitate grasping by closure of the cover 46 onto the base portion 48, it may be preferred to add teeth 50 on the edge of the base portion 48 that correspond to recesses 51 on the cover 46 (as best seen in FIG. 4).

Another alternative, included in the dispenser of FIG. 7, but applicable to other embodiments as well, is to use a ratcheting mechanism 60, shown diagramatically here but well known in the mechanical arts, that allows only one transdermal/transmucosal patch at a time to be removed. Typically, such mechanism involves a detent and pawl arrangement that allows the mechanism to turn a certain distance until it catches and prevents further movement until tension is released and then re-applied. FIG. 7 shows a dispenser 70 which includes a roll 72 containing a plurality of separably interconnected transdermal/transmucosal patches 74, on a continuous release liner 76, preferably perforated between each patch to facilitate separation. When the end of roll 72 is pulled by a user, the ratcheting mechanism 60 allows the roll to rotate until a single patch has been dispensed, upon which the patch can be separated and removed. The dispenser may also include a counter 62 (shown diagramatically, but well known in the mechanical arts) to indicate, for example, the number of patches used or remaining, or the days of the week. The counter 62 may be a separate mechanism or integrated as part of the ratcheting mechanism 60, as is well known in the mechanical arts.

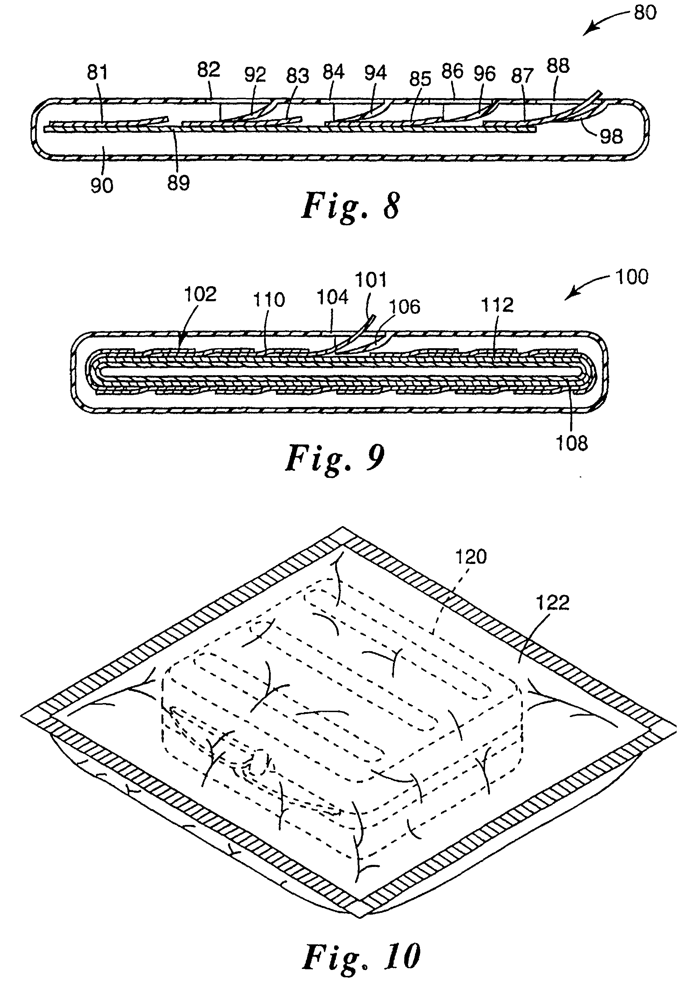

FIG. 8 shows a cross-section of another dispenser wherein the dispenser 80 includes four slots 82, 84, 86, 88, one slot for each of four separate transdermal/transmucosal patches 81, 83, 85, 87 to be dispensed. Once again, it will be understood that, although shown as a single layer for convenience, the transdermal/transmucosal patches are multi-layer articles, typically including a backing, at least one adhesive layer, and a release liner. The transdermal/transmucosal patches 81, 83, 85, 87 are all partially adhered at one end to a common carrier sheet 89 within the dispenser chamber 90. As discussed above, they may be adhered either by separate portions of adhesive or by a discontinuous release liner.

The leading portion of the transdermal/transmucosal patches 81, 83, 85, 87 is not adhered to the carrier sheet 89 to allow it to extend partly out of the dispenser slots 82, 84, 86, 88. Guide members 92, 94, 96, 98 are associated with each of the slots 82, 84, 86, 88, respectively, which guide each respective transdermal/transmucosal patch 81, 83, 85, 87 to the proper slot for removal. In the embodiment shown, the transdermal/transmucosal patches 81, 83, 85, 87 are positioned relative to the slots 82, 84, 86, 88 such that the first patch 87 to be dispensed is partially protruding from the dispenser slot 88. The other patches 81, 83, 85 do not initially protrude, but are sequentially spaced so that each one will be exposed upon removal of the preceding patch. In other words, when the first transdermal/transmucosal patch 87 is removed it will cause the carrier 89 to slide forward and thereby expose the leading portion of the next patch 85, and so on. Alternatively, the dispenser may be designed so that a portion of all of the patches initially protrudes from their respective slots, in which case they are simply grasped and removed by the user as needed.

FIG. 9 shows a dispenser 100 having a plurality of transdermal/transmucosal drug-containing patches 102 adhered on a carrier member 112 in a loop around an inner member 108. In the embodiment shown, the plurality of transdermal/transmucosal patches 102 are adhered to the carrier member 108 (e.g., by an added adhesive portion or discontinuous release liner, as previously described) which slides around the inner member 108 as the successive patches are removed. The carrier member 112 is preferably made of release liner type material, and the inner member is preferably made of a cardboard type material or the like.

A portion of the first transdermal/transmucosal patch 101 protrudes out of slot 104. A guide member 106 facilitates positioning of successive patches out of the slot 104. When the first patch 101 is grasped and removed it will partially rotate the carrier member 112 and pull the next patch 110 into position extending partly out of slot 104 for subsequent removal.

Turning now to FIG. 10, in a further aspect of the invention, it is preferred in many instances to enclose the dispenser 120 in an impermeable pouch 122 made, for example, of a metal foil and polymer material. This provides a two stage storage system for the transdermal/transmucosal drug-containing patches which allows for long term storage, but does not require individual pouches for each patch because the dispenser 120 is sufficient for (at least) short term storage of the transdermal/transmucosal patches after the pouch 122 is opened. This avoids the problems associated with the conventional method of individually pouching transdermal/transmucosal patches without sacrificing long term storage stability of the patches.

### Example

As an example of the present invention, dispensers of the design generally as in FIGS. 1 and 2 were molded out of polypropylene (Huntsman PP P6M5-008) material. Thirty 20 cm² transdermal testosterone-containing patches were loaded into the dispensers in a folded and stacked configuration as shown in FIG. 5. The patches were constructed of (i) a backing material (Scotchpak™ 1012), (ii) coated on one side with a drug, excipient, and adhesive composition comprising by weight (calculated theoretical) about 3.837% testosterone, 9.121% lauroglycol, 8.858% tetraglycol, and 10.185% terpineol mixed into 67.998% 73:7:20 isooctylacrylate acrylamide vinylacetate copolymer adhesive, and (iii) with a conventional silicon-coated 2 mil polyester release liner applied over the drug/excipientladhesive layer.

The dispensers were found to function properly, i.e., the testosterone patches could be conveniently grasped and removed sequentially. Also, stability tests were conducted to show that reasonable stability of the transdermal patches could be achieved under approximately normal, room temperature conditions of 25°C and 60% relative humidity. Moreover. even under accelerated test conditions of 40°C and 75% relative humidity the patches remained fairly stable. The individual dispensers were kept in a multilaminate foil/polymer packaging (i.e. polyester film/ethylene copolymer/aluminum foil/adhesive/Barex™ 210 film (LC flex 81703 from Smurfit Flexible Packaging, Schaumberg, IL)) until the point where the patches were to be removed on a daily basis for analysis. For comparison, testing was conducted with dispensers open and closed, and control patches were individually packaged in a conventional foil/polymer laminate packaging. Individual patches were removed daily and analyzed for testosterone and excipient (terpineol, lauroglycol, and tetraglycol) content. The results are summarized below in Tables 1-4, with the testosterone and excipient contents (weight percentage based on the total weight of the drug/excipient/adhesive layer) listed as averages over thirty day increments. The percent difference between the "0-30 days" average and "61-90 days" average is also given.

**Table 1**

| Testosterone average content | | | | |
|---|---|---|---|---|
| | 0-30 days | 31-60 days | 61-90 days | % difference |
| Control Room Temp. | 3.79 | 3.68 | 3.78 | -0.3% |
| Open Room Temp. | 3.80 | 3.79 | 3.80 | 0.0% |
| Closed Room Temp. | 3.80 | 3.81 | 3.79 | -0.3% |
| Control Accelerated | 3.72 | 3.71 | 3.63 | -2.5% |
| Open Accelerated | 3.79 | 3.65 | 3.59 | -5.3% |
| Closed Accelerated | 3.76 | 3.63 | 3.52 | -6.4% |

**Table 2**

| Terpineol average content | | | | |
|---|---|---|---|---|
| | 0-30 days | 31-60 days | 61-90 days | % difference |
| Control Room Temp. | 10.17 | 10.25 | 10.30 | -1.2% |
| Open Room Temp. | 9.95 | 9.65 | 9.82 | -1.4% |
| Closed Room Temp. | 9.96 | 9.86 | 10.04 | +0.8% |
| Control Accelerated | 10.17 | 9.66 | 9.50 | -6.6% |
| Open Accelerated | 8.64 | 7.66 | 6.72 | -22.3% |
| Closed Accelerated | 9.52 | 8.39 | 8.00 | -16.0% |

**Table 3**

| Lauroglycol average content | | | | |
|---|---|---|---|---|
| | 0-30 days | 31-60 days | 61-90 days | % difference |
| Control Room Temp. | 8.49 | 8.45 | 8.45 | -0.5% |
| Open Room Temp. | 8.47 | 8.50 | 8.54 | +0.8% |
| Closed Room Temp. | 8.49 | 8.46 | 8.45 | -.05% |
| Control Accelerated | 8.48 | 8.41 | 8.38 | -1.2% |
| Open Accelerated | 8.49 | 8.43 | 8.58 | +2.1% |
| Closed Accelerated | 8.48 | 8.41 | 8.41 | -0.9% |

**Table 4**

| Tetraglycol average content | | | | |
|---|---|---|---|---|
| | 0-30 days | 31-60 days | 61-90 days | % difference |
| Control Room Temp. | 8.58 | 8.87 | 8.77 | +2.2% |
| Open Room Temp. | 8.54 | 8.80 | 8.70 | +1.8% |
| Closed Room Temp. | 8.62 | 8.88 | 8.77 | +1.7% |
| Control Accelerated | 8.63 | 8.90 | 8.97 | +3.9% |
| Open Accelerated | 8.45 | 8.65 | 8.62 | +2.0% |
| Closed Accelerated | 8.60 | 8.93 | 9.02 | +4.8% |

The particular transdermal patch formulation used in the above testing, having moderate levels of three different excipients (terpineol, lauroglycol, and tetraglycol) in addition to testosterone, was selected in part to allow a comparison between multiple different excipients. As can be seen, the terpineol appeared to be the most unstable component of the formulation in terms of amount lost over time. Factors which may influence the amount of loss of components include such things as the type of container material used (since the material may absorb or be permeable to a component), the amount of head space in the chamber, and the effectiveness of the seal around the lid of the dispenser. The above testing confirms, however, that achieving suitable stability results with a dispenser of the present invention is indeed feasible.

As can be seen from the above, a dispenser for conveniently holding and dispensing a plurality of transdermal/transmucosal patches has been provided. It will be understood, however, that the invention has been described herein in terms of preferred embodiments and there is no intent to limit the invention to such embodiments. To the contrary, those skilled in the art will recognize that many variations of the disclosed embodiments may be practiced within the scope of the contemplated invention. Further, it should be understood that various features discussed in connection with one preferred embodiment will also be applicable to the other embodiments of the invention. For example, the desiccant, indicator strip, sealing cover, and ratcheting and/or counter mechanism will be understood by those skilled in the art to be broadly applicable to the various embodiments of the invention with appropriate modifications.

## Claims

1. A transdermal/transmucosal patch dispenser (10;120), comprising:
a chamber (26) defined by walls made of an impermeable material;
a plurality of drug-containing transdermal/transmucosal patches (20;30;31) contained within said chamber (26);
an aperture (18) through which said transdermal/transmucosal patches may be withdrawn from said chamber,
wherein said transdermal/transmucosal patches (20;30;31) are separably interconnected by a portion of adhesive (32;33) adjacent alternating ends of the transdermal/transmucosal patches, and wherein said chamber (26) is located in a base portion (14) of the dispenser, and wherein said dispenser (10) further includes a cover (12) sealing the aperture (18) and a portion of a transdermal/transmucosal patch (20) protruding from the aperture (18) from exposure to environmental contaminants.

2. The transdermal/transmucosal patch dispenser (10;120) of claim 1, further including a desiccant (34) in order to prevent contamination of the transdermal/transmucosal patches by moisture.

3. The transdermat/transmucosal patch dispenser (10;120) of claim 1, further including a moisture exposure indicator strip (28) to monitor whether or not the transdermal/transmucosal patches have been exposed to an unacceptable amount of moisture during storage.

4. The transdermal/transmucosal patch dispenser (10;120) of claim 1, further including a sealed impermeable packaging material (122) enclosing said dispenser.

5. The transdermal/transmucosal patch dispenser (10;120) of claim 1, wherein said dispenser contains at least a one month supply of about thirty patches.

6. The transdermal/transmucosal patch dispenser (10;120) of claim 1, wherein said patches contain at least one drug selected from the group consisting of estradiol, progestin, and testosterone.

7. The transdermal/transmucosal patch dispenser (10;120) of claim 1, wherein said patches contain the drug nitroglycerin.

8. The transdermal/transmucosal patch dispenser (10;120) of claim 1, wherein said patches are nonuniform and are to be delivered in a predetermined sequence established by the order in which the patches are disposed within said dispenser.

9. The dispenser (10;120) of claim 8, wherein said patches contain varying amounts of drug.

## Patentansprüche

1. Transdermal/transmukosaler Pflasterspender (10; 120) mit:
einer Kammer (26), die durch Wände gebildet ist, die aus einem undurchlässigen Material bestehen;
mehreren transdermal/transmukosalen Medikamentenpflastern (20; 30; 31), die in der Kammer (26) vorgesehen sind;
einer Öffnung (18), durch die die transdermal/transmukosalen Pflaster aus der Kammer genommen werden können,
wobei die transdermal/transmukosalen Pflaster (20; 30; 31) durch einen Klebstoffabschnitt (32; 33) im Bereich von abwechselnden Enden der transdermal/transmukosalen Pflaster trennbar miteinander verbunden sind und wobei die Kammer (26) in einem Basisabschnitt (14) des Spenders liegt und wobei der Spender (10) ferner einen Deckel (12) aufweist, der die Öffnung (18) und einen Abschnitt eines transdermal/transmukosalen Pflasters (20), der aus der Öffnung (18) vorsteht, gegen die Einwirkung kontaminierender Substanzen aus der Umgebung abdichtet.

2. Transdermal/transmukosaler Pflasterspender (10; 120) nach Anspruch 1, ferner mit einem Trockenmittel (34), um eine Kontamination der transdermal/transmukosalen Pflaster durch Feuchtigkeit zu verhindern.

3. Transdermal/transmukosaler Pflasterspender (10; 120) nach Anspruch 1, ferner mit einem Feuchtigkeitsanzeigestreifen (28), um zu überwachen, ob die transdermal/transmukosalen Pflaster während der Aufbewahrung einer nichtakzeptablen Feuchtigkeitsmenge ausgesetzt waren oder nicht.

4. Transdermal/transmukosaler Pflasterspender (10; 120) nach Anspruch 1, ferner mit einem verschlossenen undurchlässigen Verpackungsmaterial (122), das den Spender umhüllt.

5. Transdermal/transmukosaler Pflasterspender (10; 120) nach Anspruch 1, wobei der Spender mindestens einen Monatsvorrat von etwa 30 Pflastern aufweist.

6. Transdermal/transmukosaler Pflasterspender (10; 120) nach Anspruch 1, wobei die Pflaster mindestens ein Medikament aufweisen, das aus der Gruppe gewählt ist, die aus Estradiol, Progestin und Testosteron besteht.

7. Transdermal/transmukosaler Pflasterspender (10; 120) nach Anspruch 1, wobei die Pflaster das Medikament Nitroglycerin aufweisen.

8. Transdermal/transmukosaler Pflasterspender (10; 120) nach Anspruch 1, wobei die Pflaster uneinheitlich sind und in einer vorbestimmten Folge abzugeben sind, die durch die Reihenfolge bestimmt wird, in der die Pflaster in dem Spender angeordnet sind.

9. Spender (10; 120) nach Anspruch 8, wobei die Pflaster verschiedene Medikamentenmengen aufweisen.

## Revendications

1. Distributeur de timbres transdermiques/transmuqueux (10 ; 120), comprenant :
une chambre (26) définie par des parois constituées d'un matériau imperméable ;
une pluralité de timbres transdermiques/transmuqueux renfermant des substances médicamenteuses (20 ; 30 ; 31) contenus au sein de ladite chambre (26) ;
une ouverture (18) via laquelle lesdits timbres transdermiques/transmuqueux peuvent être extraits de ladite chambre,
dans lequel lesdits timbres transdermiques/transmuqueux (20 ; 30 ; 31) sont reliés les uns aux autres avec possibilité de séparation par une partie d'adhésif (32 ; 33) adjacente aux extrémités alternées des timbres transdermiques/transmuqueux, et dans lequel ladite chambre (26) est située dans une partie de base (14) du distributeur, et dans lequel ledit distributeur (10) comprend également un couvercle (12) isolant l'ouverture (18) et une partie d'un timbre transdermique/transmuqueux (20) faisant saillie depuis l'ouverture (18) d'une exposition à des agents de contamination extérieurs.

2. Distributeur de timbres transdermiques/transmuqueux (10 ; 120) selon la revendication 1, comprenant également un produit de dessiccation (34) afin d'empêcher une contamination des timbres transdermiques/transmuqueux par de l'humidité.

3. Distributeur de timbres transdermiques/transmuqueux (10 ; 120) selon la revendication 1, comprenant également une bande d'indication d'exposition à l'humidité (28) pour contrôler si les timbres transdermiques/transmuqueux ont été exposés ou non à une quantité d'humidité inacceptable durant leur stockage.

4. Distributeur de timbres transdermiques/transmuqueux (10 ; 120) selon la revendication 1, comprenant également un matériau d'emballage imperméable, étanche (122) enfermant ledit distributeur.

5. Distributeur de timbres transdermiques/transmuqueux (10 ; 120) selon la revendication 1, dans lequel ledit distributeur contient une provision pour au moins un mois d'environ trente timbres.

6. Distributeur de timbres transdermiques/transmuqueux (10 ; 120) selon la revendication 1, dans lequel lesdits timbres renferment au moins une substance médicamenteuse sélectionnée dans le groupe consistant en oestradiol, progestatif, testostérone.

7. Distributeur de timbres transdermiques/transmuqueux (10 ; 120) selon la revendication 1, dans lequel lesdits timbres renferment une substance médicamenteuse de nitroglycérine.

8. Distributeur de timbres transdermiques/transmuqueux (10 ; 120) selon la revendication 1, dans lequel lesdits timbres sont non-uniformes et doivent être distribués en une séquence prédéterminée établie par l'ordre dans lequel les timbres sont disposés au sein dudit distributeur.

9. Distributeur (10 ; 120) selon la revendication 8, dans lequel lesdits timbres renferment diverses quantités de substance médicamenteuse.
